(19) **Europäisches Patentamt** / **European Patent Office** / **Office européen des brevets**

(11) **EP 4 395 733 B1**

(12) **EUROPEAN PATENT SPECIFICATION**

(45) Date of publication and mention of the grant of the patent:
**30.04.2025 Bulletin 2025/18**

(21) Application number: **22768762.1**

(22) Date of filing: **23.08.2022**

(51) International Patent Classification (IPC):
*A61K 8/25* (2006.01)    *A61Q 17/04* (2006.01)
*A61K 8/895* (2006.01)    *A61K 8/86* (2006.01)
*A61K 8/36* (2006.01)    *A61K 8/34* (2006.01)

(52) Cooperative Patent Classification (CPC):
**A61Q 17/04; A61K 8/25; A61K 8/345; A61K 8/361; A61K 8/86; A61K 8/895**

(86) International application number:
**PCT/EP2022/073446**

(87) International publication number:
**WO 2023/030963 (09.03.2023 Gazette 2023/10)**

(54) **A PHOTOPROTECTIVE PERSONAL CARE COMPOSITION**

KÖRPERPFLEGEZUSAMMENSETZUNG MIT LICHTSCHUTZ

COMPOSITION DE SOINS PERSONNELS PHOTOPROTECTRICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2021 EP 21194673**

(43) Date of publication of application:
**10.07.2024 Bulletin 2024/28**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO SE SI SK SM**
• **Unilever Global IP Limited**
**Wirral, Merseyside CH62 4ZD (GB)**
Designated Contracting States:
**CY DE GB IE IT MT RS TR**

(72) Inventors:
• **KULKARNI, Aditi Jayavant**
**6708 WH Wageningen (NL)**
• **KUNDU, Pooja Bhupesh**
**6708 WH Wageningen (NL)**
• **LAHORKAR, Praful Gulab Rao**
**6708 WH Wageningen (NL)**
• **PAWAR, Ankita Rutu**
**6708 WH Wageningen (NL)**
• **PERUMAL, Rajkumar**
**6708 WH Wageningen (NL)**
• **VAIDYA, Ashish Anant**
**6708 WH Wageningen (NL)**

(74) Representative: **James, Helen Sarah**
**Unilever Patent Group**
**Bronland 14**
**6708 WH Wageningen (NL)**

(56) References cited:
**EP-A2- 1 281 390**    **FR-A1- 2 909 556**
**US-A1- 2004 228 888**    **US-A1- 2015 010 601**

Remarks:
The file contains technical information submitted after the application was filed and not included in this specification

**Description**

**Field of the invention**

The present invention relates to a photoprotective personal care composition, which provides high sun-protection factor and which is photostable along with very good sensorial properties that the consumer expects when such a composition is applied on the skin.

**Background of the invention**

Solar radiation includes about 5% ultraviolet (UV) radiation, wavelength of which is between 200 nm and 400 nm. It is further divided into three regions: from 320 to 400 nm (UV-A), 280 to 320 nm (UV-B) and from 200 to 280 nm (UV-C). Substantial portion of UV-C radiation is absorbed by ozone. Scientific studies have indicated that exposure to UV-A and UV-B radiation for short period causes pigmentation, reddening of the skin and localized irritation, whereas continued or prolonged exposure can lead to sunburn, melanoma, formation of wrinkles and age spots. UV radiation is also thought to cause significant damage to hair. Therefore, it is desirable to protect the skin and other keratinous substrates of the human body from the harmful effects of both UV-A and UV-B radiation. This is delivered though cosmetic compositions which provide high SPF (Sun Protection Factor).

Various cosmetic preparations are reported for preventing and/or protecting the skin from harmful effects of ultraviolet radiation. Numerous organic sunscreen agents capable of absorbing UV-A rays are reported in the field of cosmetics amongst which a particularly useful sunscreen is t-butylmethoxydibenzoylmethane (also known as Avobenzone and sold as Parsol® 1789). A variety of UV-B sunscreens are used in personal care compositions for protection from UV-B radiation. Many cosmetic manufacturers prefer to include UV-A as well as UV-B sunscreens in photoprotective compositions to provide protection over the entire range of UV radiation.

One such patent from the present applicants is published as EP2575748B1 which discloses a composition which delivers high SPF. This publication only discloses use of certain oil soluble sunscreens. In addition, patent application US 2015/010601 A1 discloses photo-protective cosmetic compositions with a good level of screening efficacy which is obtained using limited contents of UV-screening agents and that have good cosmetic properties on application.

The efficacy of sun-protection in this patent is enhanced through inclusion of certain non-ionic surfactants in combination with fatty acid and optionally certain specific polymers. With the advent of many water-soluble sunscreens, the present inventors experimented with a combination of oil-soluble and water-soluble sunscreens in order to deliver better SPF. During the course of extensive experimentation, the present inventors realized that delivery of high SPF with this approach while ensuring photostability and at the same time satisfying the consumer with desired sensory feel when applying the composition on the skin, was a difficult task. One finding during the course of this work was that when water soluble sunscreens were included in the composition the desired high SPF could be achieved only by inclusion of certain humectants. This high SPF ensured that the skin is protected from darkening, and that blemishes and dark spots are reduced.

However, the composition so prepared was seen to have poor photostability and also have poor sensory. By good sensory is meant that the composition gives a good moisturizing feel to the skin without being too oily. In other words the consumers expect a moist feel on the skin while enjoying a matte (non-oily) appearance. Further, they expect other attributes like ease of spreading and a mild and gentle feel on the skin. Having ensured the high SPF, in the composition having both water soluble as well as oil soluble sunscreens, through inclusion of certain non-ionic surfactants, fatty acids and certain humectants, the inventors were still struggling with the desired sensorial attributes. After experimenting with several choices they hit up on certain specific class of polymers that not only ensures that the high SPF is maintained but the desired sensorial attributes are achieved.

It is thus an object of the present invention to provide for a photoprotective personal care composition comprising both oil soluble and water-soluble sunscreens that not only gives high SPF but is also photostable.

It is another object of the present invention to provide for a photoprotective personal care composition which is photostable, gives high SPF, and also delivers the desired sensorial attributes when applied on the skin like moisturization, matte feel, easy spreadabiliy and a mild and gentle feel on the skin.

**Summary of the invention**

The first aspect of the present invention relates to a photoprotective composition comprising:

(i) 0.1 to 10 wt% oil-soluble sunscreen;
(ii) 0.2 to 5 wt% water-soluble sunscreen;
(iii) 0.1 to 5 wt% non-ionic surfactant having an HLB value in the range of 9 to 20;

(iv) 3 to 25 wt% fatty acid;
(v) 0.4 to 10 wt% of at least one humectant; and
(vi) 0.25 to 5 wt% of a polymer selected from one or more of (a) dimethicone/ vinyldimethicone crosspolymer and b) hydrophobically modified silica.

Another aspect of the present invention relates to a method of providing photoprotection with a in-vitro Sun Protection Factor (SPF) of at least 15 comprising a step of applying a composition of the first aspect on to the desired skin surface.

**Detailed description of the invention**

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention. Similarly, all percentages are weight/weight percentages unless otherwise indicated. Numerical ranges expressed in the format "from x to y" are understood to include x and y. When for a specific feature multiple preferred ranges are described in the format "from x to y", it is understood that all ranges combining the different endpoints are also contemplated.

The photoprotective composition of the invention is meant to be used for personal care or for cosmetic use and could also be referred to as a personal care composition or a cosmetic composition. By a "personal care composition" as used herein, is meant to include a composition for topical application to sun-exposed areas of the skin and/or hair of humans. Such a composition may be classified as leave-on or rinse off, and includes any product applied to a human body for improving appearance, cleansing, odour control or general aesthetics. The composition of the present invention can be in the form of a liquid, lotion, cream, foam, stick or gel. Non-limiting examples of such sunscreen compositions include leave-on skin lotions, creams, antiperspirants, deodorants, foundations, mascara, sunscreen lotions and wash-off shampoos, conditioners and shower gels. The composition of the present invention is preferably a leave-on composition.

"Skin" as used herein is meant to include skin on the face and body (e.g., neck, chest, back, arms, underarms, hands, legs and scalp) and especially to the sun exposed parts thereof. Compositions of the invention is also of relevance to applications on any other keratinous substrates of the human body other than skin e.g. hair where products may be formulated with specific aim of improving photoprotection.

The compositions of the invention comprise 0.1 to 10 % by weight oil-soluble sunscreen. By oil-soluble is meant those organic sunscreens which have a solubility in water of less than 10 g/l, preferably less than 5 g/l, more preferably less than 2 g/l. Oil-soluble sunscreens could be of the UV-B type or of the UV-A type. It is preferred that compositions in accordance with the invention comprise 1 to 8 % by weight of such sunscreens.

When an oil soluble UVB sunscreen is included, it is preferably one or more of octyl salicylate, 3,3,5-trimethylclohexyl 2-hydroxybenzoate, ethylhexyl salicylate, 2-ethylhexyl 2-cyano-3,3-diphenyl-2-propenoate which is also known as octocrylene, 2-ethylhexyl-4-methoxycinnamate or octylmethoxycinnamate, ethylhexyl triazone, 3-benzylidene camphor, cinoxate, diethylhexyl butamido triazone, para amino benzoic acid (PABA), ethyl dihydroxypropyl PABA, ethylhexyl dimethoxy benzylidene dioxoimidazoline propionate, isoamyl p-methoxycinnamate, isopentyl trimethoxycinnamate trisiloxane, isopropyl benzyl salicylate, isopropyl methoxycinnamate, 4-methylbenzylidene camphor, PEG-25 PABA, and pentyl dimethyl PABA. More preferred oil-soluble UVB sunscreens are selected from one or more of octyl salicylate, 3,3,5-trimethylclohexyl 2-hydroxybenzoate, ethylhexyl salicylate, 2-ethylhexyl 2-cyano-3,3-diphenyl-2-propenoate, and 2-ethylhexyl-4-methoxycinnamate and further more preferred are selected from one or more of 2-ethylhexyl-4-methoxycinnamate, ethylhexyl salicylate and 2-ethylhexyl 2-cyano-3,3-diphenyl-2-propenoate. Some of the well known brands under which the above sunscreens are sold are Octisalate®, Homosalate®, Neo Heliopan®, Neo Heliopan® AV, Neo Heliopan® OS, Octocrylene® and Parsol® MCX. The oil-soluble UV-B sunscreen has λmax from 280 to 320 nm.

Composition of the invention may comprise an organic oil-soluble UVA sunscreen. When included, it is selected from one or more of a dibenzoylmethane compound, diethylamino hydroxybenzoyl hexyl benzoate and methyl anthranilate. A dibenzoyl methane compound is most preferred for an oil-soluble UVA-sunscreen. Suitable dibenzoyl methane compounds are selected from one of more of t-butylmethoxy dibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane and 2,6-dimethyl-4-tert-butyl-4'-methoxy-dibenzoylmethane. The most preferred dibenzoyl methane compound is t-butylmethoxy dibenzoylmethane. Commercially available as Parsol 1789.

Composition of the invention may comprise both an organic oil-soluble UVB and an organic UVA sunscreen. Alternately, there are certain oil soluble sunscreens which have both a UVA and UVB screening efficacy. It is selected from one or more bis-ethylhexyloxyphenol methoxyphenyl triazine, drometrizole trisiloxane, benzophenone and its derivatives. The most preferred one is bis-ethylhexyloxyphenol methoxyphenyl triazine, commercially available as Tinosorb S.

The composition of the invention comprises 0.2 to 5 wt% of a water soluble sunscreen. By water soluble sunscreen, whether of the UVA type, or of the UVB type, is meant that the solubility in water of the sunscreen is higher than 10 g/l preferably higher than 50 g/l at 25 °C.

The preferred water-soluble UVB sunscreen for use in the present invention is phenyl benzimidazole sulphonic acid (PBSA), benzophenone-4 or benzylidene camphor sulfonic acid, preferably PBSA. PBSA also known as Ensulizole is commercially available as Eusolex 232 (from Merck KGaA). PBSA is also available under the brand names Neo Heliopan Hydro (from Symrise), Parsol HS (from DSM) and Sunsafe ES (from Uniproma). There are certain water soluble sunscreens which have both a UVA screening efficacy as well as a UVB screening efficacy. Such sunscreens provide both UVA and UVB protection e.g. benzophenone-4 (sold as Sulisobenzone). However for the purposes of the present invention benzophenone- 4 may be included as a water-soluble UV-B sunscreen.

The preferred UVA sunscreen for use in the present invention is one or more of disodium phenyl dibenzimidazole tetrasulfonate (Neoheliopan AP) or terephthalylidene dicamphor sulfonic acid (TDSA). Di sodium phenyl dibenzimidazole tetra sulfonate is also known as bisdisulizole disodium. This is commercially available as Neo heliopan AP (from Symrise Shanghai Ltd) or as Sunsafe DPDT (from Uniproma)

Terephthalylidene dicamphor sulfonic acid (TDSA) is also known as Ecamsule . This is commercially available as Mexoryl SX (US4585597) by L' Oreal or Sunsafe TDSA (from Uniproma). It can be used as parent acid or its salts to deliver the desired benefit.

The composition preferably comprises 0.1 to 5 wt%, more preferably 1 to 3 wt% water soluble UVA sunscreen. The composition preferably comprises 0.1 to 5 wt%, more preferably 1 to 4 wt%, water soluble UVB sunscreen.

The water-soluble sunscreens for inclusion in the composition of the present invention are generally commercially available in the acid form. When included in the composition in the acid form (pre neutralized forms) i.e having sulphonic acid group ($-SO_3H$) the composition additionally comprises a neutralising agent to convert the acid form in to the salt form, in which form it is known to be active as a sunscreen with an exception to Mexoryl SX which can act as sunscreen with and without neutralization. When included, the neutralising agent is preferably in 0.05% to 4% more preferably 0.2% to 2% by weight of the composition. The neutralising agent is preferably an inorganic or an organic alkali. Organic alkali is preferably an amine such as triethanol amine or diethanol amine. The present inventors have observed that the inorganic alkali is especially preferred. Preferred are alkali metal hydroxides. Most preferred metal hydroxide for inclusion as neutralising agent in the composition of the invention are sodium hydroxide or potassium hydroxide.

The composition of the invention comprises a non-ionic surfactant having an HLB value in the range of 9 to 20. More preferably the nonionic surfactant is selected from those having HLB value in the range 12 to 20, further more preferably 12 to 18.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20.

Preferably, the nonionic surfactant having HLB value in the range 9 to 20 is selected from one or more of fatty alcohol ethoxylates, fatty acid ethoxylate, alkyl phenol ethoxylates, and polyoxyethylene sorbitan alkyl esters.

Examples of fatty alcohol ethoxylates that may be used as nonionic surfactants in the composition include polyoxyethylene lauryl ether (HLB= 16.9; commercially available as Brij® 35), polyoxyethylene (20) cetyl ether (HLB=16; commercially available as Brij® 58), polyethylene glycol octadecyl ether (HLB= 18.8; commercially available as Brij® 700) and Laureth - 9 (C12EO9; HLB=14.3; commercially available as Brij® L9).

Examples of alkyl phenol ethoxylates that may be used as nonionic surfactant in the composition include octylphenol ethoxylate (HLB=15.5; commercially available as Triton™ X165), octylphenol ethoxylate (HLB=17.6; commercially available as Triton™ X405) and octylphenol ethoxylate (HLB=18.4; commercially available as Triton™ X705).

Examples of polyoxyethylene sorbitan alkyl esters that may be used as the nonionic surfactant in the composition include polyoxyethylenesorbitan monolaurate (HLB=13.3; commercially available as Tween® 21), polyoxyethylenesorbitan monolaurate (HLB=16.7; commercially available as Tween® 20), Polyoxyethylenesorbitan monopalmitate (HLB=15.6; commercially available as Tween® 40) and polyoxyethylene sorbitan monostearate (HLB= 14.9; commercially available as Tween® 60).

Fatty acid ethoxylates are available under the Myrj class. Suitable examples of commercially available non-ionic surfactants for use in the composition of this class are Myrj S40 (PEG-40 stearate), Myrj S50 (PEG-50 stearate), or Myrj 59 (PEG-100 stearate).

Preferably, the composition comprises 0.2 to 5 wt%, more preferably 0.5 to 4 wt%, even more preferably from 1 to 3 wt% nonionic surfactant having HLB in the range 9 to 20.

Humectants of the polyhydric alcohol-type can be employed as cosmetically acceptable carriers. Preferably the humectant is at least one of propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, glycerol, ethoxylated glycerol, and propoxylated glycerol. Preferred humectants are selected from one or more of glycerol, sorbitol, propylene glycol and 1,3-butylene glycol. Glycerol is the most preferred humectant. It is preferred that the amount of humectant is 1 to 10 %, more preferably 2 to 8%, further more preferably 2 to 5% by weight of the composition.

The composition of the invention comprises 0.25 to 5 wt% of a polymer selected from one or more of (a) dimethicone/ vinyldimethicone crosspolymer and b) hydrophobically modified silica. The dimethicone/ vinyldimethicone crosspolymer could be with or without silica. Various polymers of the above types could be used in the present invention. Dimethicone/ vinyl dimethicone crosspolymer which is a preferred polymer as per the invention is available as KM-9729, DOWSIL™EP-9215, SeraSnowSP 13, DOWSIL™9506 Powder, SpecsilSPD. An even more preferred polymer as per the invention is Dimethicone/Vinyl Dimethicone Crosspolymer (and) Silica which is available as DOWSIL™9701 Cosmetic Powder. Other suitable polymers which may be used are listed below:

Dimethicone/Vinyl Dimethicone Crosspolymer(and) Silica (and) Butylene Glycol available as DOWSIL™EP-9801 Hydro Cosmetic Powder

Dimethicone (and) Dimethicone/Vinyl Dimethicone Crosspolymer(and) Dimethicone Crosspolymer(and) Beeswax (and) Silica (and) Silica Silylate available as DOWSIL™9576 Smooth Away Elastomer

Dimethicone/Vinyl Dimethicone Crosspolymer(and) Polymethylsilsesquioxane(and) Silica Dimethyl Silylate Available as SSP100

Dimethicone/Vinyl Dimethicone Crosspolymer(and) LauroylLysine available as DOWSIL™EP-9289 LL

Hydrophobically modified silica is preferably selected from Silica Silylate available as DOWSIL™VM-2270 AerogelFine Particles, HDK® H2000 and

Silica dimethyl silylate available as HDK® H15, HDK® H20, HDK® H30, HDK® H18

The polymer is included in 0.25 to 5%, preferably 0.5 to 4%, more preferably 1 to 3% by weight of the composition.

The composition comprises 3 to 25 wt% fatty acid. The fatty acid is preferably a C10 to C22 fatty acid, more preferably a C16 to C18 fatty acid. Most preferably the fatty acids are stearic acid or palmitic acid or a mixture thereof. The fatty acid is often hystric acid which is substantially (generally about 90 to 95 %) a mixture of stearic acid and palmitic acid. Preferred hystric acid are ones having 40 -65% C16 and 34 -58% C18; or ones having 52 -58% C16 and 40 to 47% C18 fatty acids.

It is particularly preferred that the composition comprises 4 to 23 %, more preferably 5 to 20 %, even more preferably 6 to 17% fatty acid by weight of the composition.

Water is preferably included in 35 to 90 %, more preferably 50 to 85 %, further more preferably 50 to 80 % by weight of the composition.

The composition may optionally include polymers as spherical microporous particles. Preferred particles of this type are of the acrylate crosspolymer type. One especially preferred particle is available as Ganzpearl 820 GMP. It is included in the composition to get a smooth creamy feel and to achieve high oil absorbency. Other polymeric particles which may be included are styrene/acrylates copolymer which are available under the brand name of SunSpheres™ Powder, SunSpheres™ PGL and SunSpheres™ LCG from Dow.

In addition to the ingredients disclosed earlier, the compositions, may and preferably do include other ingredients in order to perform one or more functions of this invention also include a cosmetically acceptable carrier. Emollient materials may serve as cosmetically acceptable carriers. These may be in the form of silicone oils, natural or synthetic esters, and hydrocarbons. Amounts of the emollients may range anywhere from 0.1 to 25 %, preferably between 1 and 10 % by weight of the composition. Silicone oils may be divided into the volatile and nonvolatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 5 to 6, silicon atoms.

Nonvolatile silicone oils useful as an emollient materials include polyalkyl siloxanes, polyalkylaryl siloxanes and polyether siloxane copolymers. The essentially nonvolatile polyalkyl siloxanes useful herein include, for example, polydimethyl siloxanes with viscosities of from $5 \times 10^{"6}$ to 0.1 m²/s at 25 °C. Among the preferred nonvolatile emollients useful in the present compositions are the polydimethyl siloxanes having viscosities from $1 \times 10^5$ to $4 \times 10^4$ m²/s at 25 °C.

Another class is of ester emollients, which are alkyl esters of saturated fatty acids having 10 to 24 carbon atoms. Examples thereof include behenyl neopentanoate, isononyl isonanonoate, isopropyl myristate and octyl stearate. Alternatively, they are ether-esters such as fatty acid esters of ethoxylated saturated fatty alcohols. Further alternatively they are polyhydric alcohol esters such as ethylene glycol mono and di-fatty acid esters, diethylene glycol mono- and di-fatty acid esters, polyethylene glycol (200-6000) mono- and di- fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C1-C30 alcohols. Yet further alternatively they are wax esters such as beeswax, spermaceti wax and tribehenin wax. Yet further alternatively they are sugar esters of fatty acids such as sucrose polybehenate and sucrose polycottonseedate. Further alternatively they are natural ester emollients principally based upon mono-, di- and tri-glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20 % by weight of the compositions.

Hydrocarbons, which are suitable cosmetically acceptable carriers include petrolatum, mineral oil, $C_8$-$C_{18}$ isoparaffins, polybutenes and especially isohexadecane, available commercially as Permethyl® 101A from Presperse Inc.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol and mixtures thereof. Thickeners can be utilized as part of the cosmetically acceptable carrier of compositions according to the present invention. Typical thickeners include crosslinked acrylates (e.g. Carbopol® 982 and Carbopol® Ultrez 10), hydrophobically-modified acrylates (e.g. Carbopol 1382(R)), polyacrylamides (e.g. Sepigel® 305), acryloylmethylpropane sulfonic acid/salt polymers and copolymers (e.g. Aristoflex® HMB and AVC), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum®). Amounts of the thickener may range from 0.0001 to 10%, usually from 0.001 to 2.5 %, optimally from 0.01 to 1 % by weight of the composition. Preferred are emollients that can be used, especially for products intended to be applied to the face, to improve sensory properties and are chosen from the group of oils that do not form stiff gels with 12HSA; these include polypropylene glycol-14 butyl ether otherwise known as Tegosoft® PBE, or PPG15 stearyl ether such as Tegosoft® E, other oils such as esters, specifically, isopropyl myristate, isopropyl palmitate, other oils could include castor oils and derivatives thereof.

The inventive compositions preferably includes a skin brightening compound. Illustrative substances are placental extract, lactic acid, niacinamide, arbutin, kojic acid, ferulic acid, hydroquinone, resorcinol and derivatives including 4-substituted resorcinols and combinations thereof. More preferably, such skin brightening compound is a tyrosinase inhibitor, to complement the melanogenesis inhibition activity of the substituted monoamines, most preferably a compound selected from the group consisting of kojic acid, hydroquinone and 4-substituted resorcinol. Also, dicarboxylic acids represented by the formula HOOC-(CxHy)-COOH where x=4 to 20 and y=6 to 40 such as azelaic acid, sebacic acid, oxalic acid, succinic acid, fumaric acid, octadecenedioic acid or their salts or a mixture thereof, most preferably fumaric acid or salt thereof, especially di-sodium salt.

Combination of 12HSA with fumaric acid or salts thereof are particularly preferred, especially for skin brightening formulations. Amounts of these agents may range from 0.1 to 10 %, preferably from 0.5 to 2 % by weight of the composition. It is preferred that the skin brightening coactive according to the invention is vitamin B3 or a derivative thereof and is selected from the group consisting of niacinamide, nicotinic acid esters, non-vasodilating esters of nicotinic acid, nicotinyl amino acids, nicotinyl alcohol esters of carboxylic acids, nicotinic acid N-oxide, niacinamide N-oxide and mixtures thereof.

Another preferred ingredient of the inventive compositions is a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., $C_2$-C22 alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. These compounds are well known in the art and are commercially available from a number of sources, e.g., Sigma Chemical Company (St. Louis, Mo.), and Boerhinger Mannheim (Indianapolis, Ind.). Preferred retinoids are retinol, retinyl palmitate, retinyl acetate, retinyl propionate, retinal and combinations thereof. The retinoid is preferably substantially pure, more preferably essentially pure.

Colorants, opacifiers and abrasives may also be included in compositions of the present invention. Each of these substances may range from 0.05 to 5 %, preferably between 0.1 and 3 % by weight of the composition.

Preservatives could be incorporated into the compositions of this invention to protect against the growth of potentially harmful microorganisms. Suitable traditional preservatives for compositions of this invention are alkyl esters of para-hydroxybenzoic acid. Other preservatives which have come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, caprylyl glycol, C1-6 parabens (especially, methyl paraben and/or propyl paraben), imidazolidinyl urea, sodium dehydroacetate and benzyl alcohol. The preservatives should be selected having regard for the use of the composition and possible incompatibilities between the preservatives and other ingredients in the emulsion.

Preservatives are preferably employed in amounts ranging from 0.01 % to 2 % by weight of the composition, including all ranges subsumed therein. An especially preferred combination is octocrylene and caprylyl glycol, since caprylyl glycol has been disclosed to enhance UVA and UVB protection. The compositions of the present invention can comprise a wide range of other optional components. The CTFA Cosmetic Ingredient Handbook, Second Edition, 1992, describes a wide variety of non-limiting cosmetic and pharmaceutical ingredients commonly used in the skin care industry, which are suitable for use in the compositions of the present invention. Examples include antioxidants, binders, biological additives, buffering agents, colorants, astringents, fragrance, opacifying agents, conditioners, exfoliating agents, pH adjusters, natural extracts, essential oils, skin sensates, skin soothing agents and skin healing agents.

The compositions of the present invention are preferably non-solid. The compositions of the invention are preferably leave-on compositions. Such compositions are applied on the skin and remain thereon for prolonged time. Rather, as leave-on compositions are not intended to be rinsed-off they need to be non-irritating and therefore it would be necessary to minimize the total level of surfactant and the total level of anionic surfactant in leave-on compositions. The total level of anionic surfactant in the inventive compositions is preferably no more than 10 %, more preferably below 8 %, most preferably at most 5 %, optimally at most 3 %.

The compositions of the present invention are preferably in a lotion format. The composition of the present invention provides Sun Protection Factor (SPF) of at least 15.

Compositions for Use in a Method

The composition according to the invention is intended primarily as a product for topical application to human skin, especially as an agent for protecting from solar radiation, and preventing or reducing pigmentation, the appearance of wrinkled or aged skin, or age spots.

In use, a small quantity of the composition, for example from 1 to 5 ml, is applied to exposed area of the skin, from a suitable container or applicator and, if necessary, it is then spread over and/or rubbed into the skin using the hand or fingers or a suitable device.

In accordance with another aspect is disclosed a composition of the invention for use in a method of providing photoprotection comprising a step of applying a composition of the invention said method provides Sun Protection Factor of at least 15, preferably at least 20, more preferably at least 25 ideally at least 30.

The individual Sun Protection Factor (SPF) for each subject is defined as the ratio of the amount of energy (dose, in units of $J/m^2$ or in seconds of exposure time) required to produce minimal erythema on protected skin to the amount of energy needed to produce minimal erythema on untreated skin calculated as follows:

$$SPF = \frac{\text{MED Protected Skin}}{\text{MED Unprotected Control Site}}$$

The SPF for the product is calculated by taking the arithmetic mean of the individual SPF values for all the subjects.

While the above summarizes the present invention, the invention will now be further illustrated in the following non-limiting examples.

**Examples**

Example A,B, 1, 2: Effect of the compositions of the invention as compared to an existing composition in delivering SPF and sensorial properties while being highly photostable.

Table 1 shows the various compositions (lotions) that were prepared.

Table - 1

| Ingredient | Example - 1 | Example - 2 | Example - A | Example - B |
|---|---|---|---|---|
| Parsol HS | 1.50 | 1.50 | 1.50 | 1.50 |
| Neo Heliopan Octylsalicylate | 4.00 | 4.00 | 5.00 | 4.00 |
| Parsol 1789 | 1.00 | 1.00 | 3.00 | 1.00 |

(continued)

| Ingredient | Example - 1 | Example - 2 | Example - A | Example - B |
|---|---|---|---|---|
| Octocrylene Neoheliopan 303 | 1.00 | 1.00 | 0.00 | 1.00 |
| Myrj 59 | 1.19 | 1.19 | 1.19 | 1.19 |
| Fatty acid mix[a] | 6.00 | 6.00 | 2.35 | 2.35 |
| Glycerin | 2.50 | 2.50 | 2.50 | 2.50 |
| DC 9701 | 1.00 | 1.00 | 0.00 | 0.00 |
| Di sodium EDTA | 0.10 | 0.10 | 0.05 | 0.10 |
| Carbopol Ultrez 10 | 0.40 | 0.40 | 0.40 | 0.40 |
| Ganzpearl 820 | 0.00 | 0.50 | 0.00 | 0.00 |
| Keltrol CG1000 Xanthum gum | 0.30 | 0.30 | 0.50 | 0.30 |
| Sodium hydroxide | 0.23 | 0.23 | 0.25 | 0.23 |
| Pottasium hydroxide 84% | 0.54 | 0.54 | 0.45 | 0.58 |
| Niacinamide | 0.10 | 0.10 | 0.10 | 0.10 |
| Glyceryl Stearate | 0.65 | 0.65 | 0.65 | 0.65 |
| Cetyl alcohol / Ginol 16 | 0.37 | 0.37 | 0.37 | 0.37 |
| Glycol stearate 97% + Stearamide AMP 3% | 1.39 | 1.39 | 1.39 | 1.39 |
| Silicon DC 200/50cst | 1.00 | 1.00 | 1.00 | 1.00 |
| Water | to 100 | to 100 | to 100 | to 100 |
| [a] The fatty acid mix was a combination of primarily palmitic acid and stearic acid in a weight ratio of about 55:45. | | | | |

The procedure for preparing the compositions of Table 1 is as follows:
The polymers Ultrez 10 and xanthan gum were predispersed in water in a separate beaker. The oil phase ingredients such as fattty acid mix, non ionic surfactant, emollients, and oil soluble sunscreens were added to a glass beaker and melted at 75°C. If needed, the sensory agents DC 9701 and Ganzpearl 820 were pre-dispersed in the molten oil phase before adding to emulsification process. The water-soluble sunscreens were pre-neutralized in a separate beaker using alkali (NaOH or KOH) in water. In the main container glycerin and water were homogenized at 75°C. The pre-neutralized water soluble sunscreens were added and homogenized. After homogenization, the molten oil phase was added to the main container and homogenization was continued. After complete mixing, the predispersd polymers were added and neutralized as required. After complete mixing, other ingredients were added and at 45°C.

The in vitro SPF and photostability values for the compositions of Table 1 are summarized in Table - 2 below. They were measured using the following procedure:
Thin film transmittance measurements were done using Labsphere's Ultraviolet transmittance analyzer. In this study, 70 x 70 mm PMMA plate with ~6 $\mu$m roughness from Schönberg GmbH & Co were used. The percent transmittance of the various compositions was measured using a procedure as outlined below. 2 mg/cm$^2$ of sample was applied on PMMA plate, distributed uniformly using a syringe, and spread uniformly. The drying time for the PMMA plates was 30 minutes. After the drying time, sample plates were exposed to UV light and transmittance scan was recorded. This scan gives the transmittance as a function of wavelength (290 - 400 nm) for a given sample. For a single plate six to nine different spots were scanned. The same was repeated for 2 or 3 plates. The data reported is thus an average over 12 -18 readings. The reference transmittance scan was obtained using a blank plate, with no sample on the PMMA plates with glycerine spread on it. The transmittance values were used to arrive at the SPF values using the UV -2000s application provided with the instrument.

Percentage of transmittance (and therefore the SPF) was measured *invitro* SPF at zero time (line 0 min). Same plate was exposed to solar simulated light and % of transmittance measured at designated time intervals from 0 and 60 min. Average absorbance at 310 & 355 nm was recorded to measure % UVB & UVA photostability respectively. UV was generated using solar simulator (Atlas kW system) using irradiance of 550 W m$^{-2}$, at a distance of ~ 20 cm.

Table - 2

|  | Example - 1 | Example - 2 | Example - A | Example - B |
|---|---|---|---|---|
| SPF | 33.5 | 37.0 | 48.8 | 21.0 |
| STDV | 0.4 | 3.2 | 6.8 | 0.8 |
| Photostability @ 355 nm after 60 min exposure | ~87% | ~86% | ~67% | ~85% |
| Note : The light exposure was done using Atlas CPS+ instrument at 550 W/m$^2$ . | | | | |

The data in Table 2 indicates that compositions as per the invention (Example 1 and 2) provides for both high SPF and high photostability which is not achieved by examples outside the invention (Example A which has low photostability) and Example B (which has low SPF).

In addition to measuring SPF, the compositions were used by a panel of consumers to evaluate the tactile sensory performance. The tests were carried out in the following manner.

The study was conducted on a total of 24 females subjects ranging in age from 25 to 45 years. They were engaged in a clinical study that evaluated the tactile performance of the test products. The study has a sequential monadic design. The study includes offline, self filled structured quantitative questionnaire and 15 - 20 minutes of interview to understand the product performance, key likes, dislikes which includes product preferences, protection of skin from darkening, reduction of blemishes/ dark spots on the face, visible reduction of appearance of spots, quick and easy absorption into skin, product spreading, moisturization, matte feel of skin and sweat proof nature. Each panellist evaluated the control sample (Example A) and the inventive samples (Examples 1 and 2) for three days each. The study population was divided into two groups/ panel and in each group 12 females were present.

The data is summarized in Table - 3

Table - 3

| Attributes | Example A | Example 1 | Example 2 |
|---|---|---|---|
| Protects skin from darkening | 38 | 75* | 50 |
| Reduces blemishes/ dark spots on the face | 17 | 50* | 42 |
| Visibly reduces appearance of spots | 17 | 58* | 42 |
| Is absorbed quickly and easily into my skin | 54 | 67 | 75 |
| Is easy to apply or spread | 58 | 83 | 92* |
| Leaves my skin feeling moisturized | 50 | 83** | 67 |
| Feels matte on my skin | 17 | 58* | 33 |
| Is sweat proof | 29 | 33 | 67* |
| *denotes significantly higher at 95% CI<br>** denotes significantly higher at 90% CI | | | |

The overall preference is summarized in Table -4 below:

Table - 4:

| Product | Example A | Example 1 | Example 2 |
|---|---|---|---|
| Panel 1 | 25 | 75* | - |
| Panel 2 | 42 | - | 58 |

The data in Table 3 and 4 indicates that compositions as per the invention (Example 1 and 2) are more preferred by consumers on many sensory attributes as compared to Example A. Of the two, Example 1 appears to be more preferred as compared to Example 2.

**Claims**

1. A photoprotective composition comprising:

    (i) 0.1 to 10 wt% oil-soluble sunscreen;
    (ii) 0.2 to 5 wt% water-soluble sunscreen;
    (iii) 0.1 to 5 wt% non-ionic surfactant having an HLB value in the range of 9 to 20;
    (iv) 3 to 25 wt% fatty acid;
    (v) 0.4 to 10 wt% of at least one humectant; and
    (vi) 0.25 to 5 wt% of a polymer selected from one or more of (a) dimethicone/ vinyldimethicone crosspolymer and b) hydrophobically modified silica.

2. A composition as claimed in claim 1 wherein said oil-soluble sunscreen is chosen from a UV-B sunscreen selected from one or more of octyl salicylate, 3,3,5-trimethylclohexyl 2-hydroxybenzoate, ethylhexyl salicylate, 2-ethylhexyl 2-cyano-3,3-diphenyl-2-propenoate, 2-ethylhexyl-4-methoxycinnamate and octylmethoxycinnamate; or a UV-A sunscreen selected from one or more of a dibenzoylmethane compound, diethylamino hydroxybenzoyl hexyl benzoate and methyl anthranilate.

3. A composition as claimed in claim 2 wherein the dibenzoylmethane compound is selected from one or more of t-butylmethoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane and 2,6-dimethyl-4-tert-butyl-4'-methoxydibenzoylmethane.

4. A composition as claimed in any one of the preceding claims wherein said water-soluble sunscreen is chosen from a UV-B sunscreen selected from one or more of 2-phenylbenzimidazole-5-sulfonic acid, benzophenone-4 and benzylidene camphor sulfonic acid ; or a water-soluble UV-A sunscreen selected from one or more of di sodium phenyl dibenzimidazole tetra sulfonate and terephthalylidene dicamphor sulfonic acid.

5. A composition as claimed in any one of the preceding claims wherein the non-ionic surfactant has an HLB value in the range of 12 to 20.

6. A composition as claimed in any preceding claims wherein the non-ionic surfactant is selected from one or more of a fatty alcohol ethoxylate, fatty acid ethoxylate, alkyl phenol ethoxylate, and polyoxyethylene sorbitan alkyl esters; preferably a fatty alcohol ethoxylate or a fatty acid ethoxylate.

7. A composition as claimed in any one of the preceding claims wherein said humectant is selected from one or more of a propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, glycerol, ethoxylated glycerol and propoxylated glycerol.

8. A composition as claimed in any one of the preceding claims wherein the fatty acid is selected from one or more of stearic acid and palmitic acid, preferably a mixture of stearic acid and palmitic acid.

9. A composition as claimed in any one of the preceding claims wherein said composition provides in-vitro Sun Protection Factor (SPF) of at least 15.

10. Composition as claimed in any one of the preceding claims for use in a method of providing photoprotection with a Sun Protection Factor (SPF) of at least 15, the method comprising a step of applying the composition on to the desired skin surface.

**Patentansprüche**

1. Zusammensetzung mit Lichtschutz, umfassend:

    (i) 0,1 bis 10 Gew.-% öllösliches Sonnenschutzmittel;

EP 4 395 733 B1

(ii) 0,2 bis 5 Gew.-% wasserlösliches Sonnenschutzmittel;
(iii) 0,1 bis 5 Gew.-% nicht-ionisches Tensid mit einem HLB-Wert in dem Bereich von 9 bis 20;
(iv) 3 bis 25 Gew.-% Fettsäure;
(v) 0,4 bis 10 Gew.-% mindestens eines Feuchthaltemittels; und
(vi) 0,25 bis 5 Gew.-% eines Polymers, ausgewählt aus einem oder mehreren der folgenden Verbindungen: (a) Dimethicon/Vinyldimethicon-Crosspolymer und b) hydrophob modifiziertem Siliziumdioxid.

2. Zusammensetzung, wie im Anspruch 1 beansprucht, wobei das öllösliche Sonnenschutzmittel ausgewählt ist unter einem UV-B-Sonnenschutzmittel, ausgewählt aus einem oder mehreren der folgenden Verbindungen: Octylsalicylat, 3,3,5-Trimethylcyclohexyl-2-hydroxybenzoat, Ethylhexylsalicylat, 2-Ethylhexyl-2-cyano-3,3-diphenyl-2-propenoat, 2-Ethylhexyl-4-methoxycinnamat und Octylmethoxycinnamat; oder unter einem UV-A-Sonnenschutzmittel, ausgewählt aus einem oder mehreren der folgenden Verbindungen: einer Dibenzoylmethan-Verbindung, Diethylaminohydroxybenzoylhexylbenzoat und Methylanthranilat.

3. Zusammensetzung, wie im Anspruch 2 beansprucht, wobei die Dibenzoylmethan-Verbindung unter einer oder mehreren der folgenden Verbindungen ausgewählt ist: t-Butylmethoxydibenzoylmethan, 2-Methyldibenzoylmethan, 4-Methyl-dibenzoylethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyl-dibenzoylmethan, 2-Methyl-5-isopropyl-4'-methoxydibenzoylmethan, 2-Methyl-5-tert-butyl-4'-methoxy-dibenzoylmethan, 2,4-Dimethyl-4'-methoxydibenzoylmethan und 2,6-Dimethyl-4-tert-butyl-4'-methoxy-dibenzoylmethan.

4. Zusammensetzung, wie in irgendeinem der vorhergehenden Ansprüche beansprucht, wobei das wasserlösliche Sonnenschutzmittel ausgewählt ist unter einem UV-B-Sonnenschutzmittel, ausgewählt aus einer oder mehreren der folgenden Verbindungen: 2-Phenylbenzimidazol-5-sulfonsäure, Benzophenon-4- und Benzylidenkampfersulfonsäure; oder unter einem wasserlöslichen UV-A-Sonnenschutzmittel, ausgewählt aus einer oder mehreren der folgenden Verbindungen: Dinatriumphenyldibenzimidazoltetrasulfonat und Terephthalylidendikampfersulfonsäure.

5. Zusammensetzung, wie in irgendeinem der vorhergehenden Ansprüche beansprucht, wobei das nicht-ionische Tensid einen HLB-Wert in dem Bereich von 12 bis 20 aufweist.

6. Zusammensetzung, wie in einem der vorhergehenden Ansprüche beansprucht, wobei das nicht-ionische Tensid unter einer oder mehreren der folgenden Verbindungen ausgewählt ist: einem Fettalkoholethoxylat, Fettsäureethoxylat, Alkylphenolethoxylat und Polyoxyethylensorbitanalkylestern, bevorzugt einem Fettalkoholethoxylat oder einem Fettsäureethoxylat.

7. Zusammensetzung, wie in irgendeinem der vorhergehenden Ansprüche beansprucht, wobei das Feuchthaltemittel unter einer oder mehreren der folgenden Verbindungen ausgewählt ist: einem Propylenglykol, Dipropylenglykol, Polypropylenglykol, Polyethylenglykol, Sorbitol, Hydroxypropylsorbitol, Hexylenglykol, 1,3-Butylenglykol, Isoprenglykol, 1,2,6-Hexantriol, Glycerin, ethoxyliertem Glycerin und propoxyliertem Glycerin.

8. Zusammensetzung, wie in irgendeinem der vorhergehenden Ansprüche beansprucht, wobei die Fettsäure unter einer oder mehreren der folgenden Verbindungen ausgewählt ist: Stearinsäure und Palmitinsäure, bevorzugt einer Mischung aus Stearinsäure und Palmitinsäure.

9. Zusammensetzung, wie in irgendeinem der vorhergehenden Ansprüche beansprucht, wobei die Zusammensetzung einen in-vitro-Sonnenschutzfaktor (SPF) von mindestens 15 zeigt.

10. Zusammensetzung, wie in irgendeinem der vorhergehenden Ansprüche beansprucht, zur Verwendung in einem Verfahren zur Bereitstellung von Lichtschutz mit einem Sonnenschutzfaktor (SPF) von mindestens 15, wobei das Verfahren einen Schritt des Auftragens der Zusammensetzung auf die gewünschte Hautoberfläche umfasst.

**Revendications**

1. Composition photoprotectrice comprenant :

(i) 0,1 à 10 % en poids d'écran solaire liposoluble ;
(ii) 0,2 à 5 % en poids d'écran solaire hydrosoluble ;

(iii) 0,1 à 5 % en poids de tensioactif non-ionique ayant un indice HLB situé dans la plage allant de 9 à 20 ;

(iv) 3 à 25 % en poids d'acide gras ;

(v) 0,4 à 10 % en poids d'au moins un humectant ; et

(vi) 0,25 à 5 % en poids d'un polymère qui est un ou plusieurs choisis parmi (a) un polymère réticulé de diméthicone/vinyldiméthicone et b) une silice modifiée de manière hydrophobe.

2. Composition selon la revendication 1, dans laquelle ledit écran solaire liposoluble est choisi parmi un écran solaire anti-UVB qui est un ou plusieurs choisis parmi le salicylate d'octyle, le 2-hydroxybenzoate de 3,3,5-trimethylcyclohexyle, le salicylate d'éthylhexyle, le 2-cyano-3,3-diphényl-2-propénoate de 2-éthylhexyle, le 4-méthocycinnamate de 2-éthylhexyle et le méthoxycinnamate d'octyle ; ou un écran solaire anti-UVA qui est un ou plusieurs choisis parmi un composé dibenzoylméthane, le benzoate de diéthylaminohydroxybenzoylhexyle et l'anthranylate de méthyle.

3. Composition selon la revendication 2, dans lequel le composé dibenzoylméthane est un ou plusieurs choisis parmi le t-butylméthoxydibenzoylméthane, le 2-méthyldibenzoylméthane, le 4-méthyldibenzoyléthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 2-méthyl-5-isopropyl-4'-méthoxydibenzoylméthane, le 2-méthyl-5-tert-butyl-4'-méthoxydibenzoylméthane, le 2,4-diméthyl-4'-méthoxydibenzoylméthane et le 2,6-diméthyl-4-tert-butyl-4'-méthoxydibenzoylméthane.

4. Composition selon l'une quelconque des revendications précédentes, dans laquelle ledit écran solaire hydrosoluble est choisi parmi un écran solaire anti-UVB qui est un ou plusieurs choisis parmi l'acide 2-phénylbenzimidazole-5-sulfonique, la benzophénone-4 et l'acide benzylidènecamphosulfonique ; ou un écran solaire anti-UVA hydrosoluble qui est un ou plusieurs choisis parmi le tétrasulfonate de phényldibenzimidazole disodique et l'acide téréphtalylidènedicamphosulfonique.

5. Composition selon l'une quelconque des revendications précédentes, dans laquelle le tensioactif non-ionique a un indice HLB situé dans la plage allant de 12 à 20.

6. Composition selon l'une quelconque des revendications précédentes, dans laquelle le tensioactif non-ionique est un ou plusieurs choisis parmi un alcool gras éthoxylé, un acide gras éthoxylé, un alkylphénol éthoxylé, et un ester alkylique de sorbitan polyoxyéthyléné ; de préférence un alcool gras éthoxylé ou un acide gras éthoxylé.

7. Composition selon l'une quelconque des revendications précédentes, dans laquelle ledit humectant est un ou plusieurs choisis parmi le propylèneglycol, le dipropylèneglycol, le polypropylèneglycol, le polyéthylèneglycol, le sorbitol, l'hydroxypropylsorbitol, l'hexylèneglycol, le 1,3-butylèneglycol, l'isoprèneglycol, le 1,2,6-hexanetriol, le glycérol, le glycérol éthoxylé et le glycérol propoxylé.

8. Composition selon l'une quelconque des revendications précédentes, dans laquelle l'acide gras est un ou plusieurs choisis parmi l'acide stéarique et l'acide palmitique, de préférence un mélange d'acide stéarique et d'acide palmitique.

9. Composition selon l'une quelconque des revendications précédentes, dans laquelle ladite composition assure un facteur de protection solaire (FPS) *in vitro* d'au moins 15.

10. Composition selon l'une quelconque des revendications précédentes, pour une utilisation dans une méthode de fourniture d'une photoprotection avec un facteur de protection solaire (FPS) d'au moins 15, la méthode comprenant une étape d'application de la composition sur la surface de peau souhaitée.

## REFERENCES CITED IN THE DESCRIPTION

**Patent documents cited in the description**

- EP 2575748 B1
- US 2015010601 A1
- US 4585597 A

**Non-patent literature cited in the description**

- The CTFA Cosmetic Ingredient Handbook. 1992